(19) **Europäisches Patentamt European Patent Office Office européen des brevets**

(11) **EP 2 480 654 B1**

(12) **FASCICULE DE BREVET EUROPEEN**

(45) Date de publication et mention de la délivrance du brevet:
**17.06.2015 Bulletin 2015/25**

(51) Int Cl.:
*C12M 3/06* *(2006.01)* *B01D 35/30* *(2006.01)*
*B81B 1/00* *(2006.01)*

(21) Numéro de dépôt: **10757102.8**

(22) Date de dépôt: **23.09.2010**

(86) Numéro de dépôt international:
**PCT/EP2010/064082**

(87) Numéro de publication internationale:
**WO 2011/036226 (31.03.2011 Gazette 2011/13)**

(54) **DISPOSITIF ET SYSTEME DE FILTRATION**

FILTRIERVORRICHTUNG UND SYSTEM

FILTRATION DEVICE AND SYSTEM

(84) Etats contractants désignés:
**AL AT BE BG CH CY CZ DE DK EE ES FI FR GB GR HR HU IE IS IT LI LT LU LV MC MK MT NL NO PL PT RO SE SI SK SM TR**

(30) Priorité: **23.09.2009 FR 0956568**

(43) Date de publication de la demande:
**01.08.2012 Bulletin 2012/31**

(73) Titulaires:
• **Université Technologie de Compiègne-UTC**
**60203 Compiegne cedex (FR)**
• **Centre National de la Recherche Scientifique (CNRS)**
**75016 Paris (FR)**

(72) Inventeurs:
• **PAULLIER, Patrick**
**F-60150 Thourotte (FR)**
• **OULD DRIS, Aïssa**
**F-60200 Compiegne (FR)**

• **LECLERC, Eric**
**F-60280 Margny Les Compiegne (FR)**

(74) Mandataire: **Regimbeau**
**20, rue de Chazelles**
**75847 Paris Cedex 17 (FR)**

(56) Documents cités:
**WO-A1-2004/020573      US-A- 5 888 807**
**US-A1- 2007 155 007      US-B1- 6 197 575**
**US-B2- 7 048 856**

• **BAUDOIN RÉGIS: "Développement et caractérisation d'une puce à cellules pour le calibrage d'agents toxiques", THESIS PRÉSENTÉE POUR L'OBTENTION DU GRADE DE DOCTEUR DE L'UTC, XX, XX, [Online] 27 novembre 2008 (2008-11-27), pages 1-61, XP002576413, Extrait de l'Internet: URL:http://tel.archives-ouvertes.fr/docs/0 0/34/23/42/PDF/Rapport_THESE_Ve rsion_finale.pdf> [extrait le 2010-04-01] cité dans la demande**

**Description**

DOMAINE TECHNIQUE GENERAL

La présente invention concerne la reproduction in vitro de phénomènes de filtration.

Plus précisément, elle concerne un dispositif de filtration pour un bioréacteur avec une membrane séparant deux chambres et un système de filtration mettant en oeuvre un ou plusieurs exemplaires d'un tel dispositif de filtration.

ETAT DE L'ART

La greffe demeure actuellement la solution la plus efficace pour le traitement des troubles hépatiques et des dysfonctionnements rénaux. Cependant, l'insuffisance de donneurs oblige les patients en attente d'organe à subir des traitements lourds et réguliers très souvent sources de complications. Un des grands enjeux de l'ingénierie des tissus réside donc dans la mise au point d'organes artificiels capables de se substituer aux organes défaillants ou absents. Les patients verraient alors leurs conditions de vie s'améliorer et le coût des soins diminuer. A cet effet, de nombreuses recherches ont été menées pour reproduire in vitro des phénomènes internes aux organes du corps humain et animal, en particulier les phénomènes de filtration.

Pour ce faire, il est nécessaire de disposer d'une part de bioréacteurs reproduisant un environnement favorable au développement et à l'organisation de cellules, proche de celui d'un tissu ou d'un organe animal ou humain, d'autre part de membranes capables de reproduire les phénomènes de filtration, par exemple, la filtration glomérulaire dans le rein. De nombreuses solutions ont été proposées dans l'art antérieur.

Le document US 6 197 575 présente un dispositif de culture de cellules pour obtenir in vitro des tissus ou organes artificiels. Ce dispositif comprend une enceinte avec une membrane de filtration séparant l'enceinte en deux chambres, membrane sur laquelle sont disposés des canaux destinés à recevoir une culture de cellules. Ainsi, la membrane jouant le rôle de filtre est également utilisée comme support de culture de cellules dans une chambre de culture, l'autre partie de l'enceinte constituant une chambre d'évacuation. Ceci laisse supposer que la membrane présente une certaine rigidité. Différentes combinaisons d'arrivée/évacuation de fluide pour les chambres de culture et d'évacuation sont envisagées (voir les figures 1 à 2c du document US 6 197 575, chaque combinaison correspondant à une utilisation spécifique du dispositif.

L'article « A MEMS-Based Renal Replacement System » publié en juin 2004 décrit une unité de traitement du sang prévue pour une application en hémodialyse continue. L'unité se compose d'un empilement de dispositifs bicouches comprenant chacun un réseau de canaux pour une circulation sanguine et une chambre d'évacuation, les deux réseaux étant en vis-à-vis et séparés par une membrane d'ultrafiltration (voir figure 2 de ce document). Les auteurs de cet article ont utilisé un algorithme pour définir la morphologie d'un réseau reproduisant les conditions de circulation du sang dans les vaisseaux sanguins humains.

Si l'unité présentée permet de mimer efficacement les conditions de transport du sang dans les vaisseaux sanguins, elle n'est pas sans poser des difficultés. En effet, le sang est confiné dans des canaux à section très étroite (hauteur de 35 microns), ce qui limite le débit de sang traité par chaque dispositif de l'entité et peut induire des problèmes d'engorgement des canaux. Les auteurs prévoient pas moins de 100 dispositifs bicouches nécessaires pour réaliser une hémodialyse.

Le brevet US 7 048 856 présente un dispositif d'ultrafiltration compact pouvant être utilisé comme un bioréacteur. Le dispositif comprend une chambre dans laquelle est disposée une membrane d'ultrafiltration, laquelle membrane sépare la chambre en une partie de filtration et une partie d'évacuation, ainsi qu'une entrée de fluide, une sortie de fluide de filtration et une sortie de fluide d'évacuation. La membrane est adaptée au fluide en entrée et à la filtration envisagée. Par exemple, la membrane peut avoir des pores dont la taille est choisie pour filtrer l'urée dans le sang. Par ailleurs, selon une réalisation particulière présentée dans ce document, le dispositif peut comprendre une chambre d'analyse dans laquelle le fluide filtré est analysé. La membrane peut aussi accueillir une culture de cellules. L'entrée et les sorties de fluide peuvent être munies de pompes ou de vannes pour contrôler le débit, éventuellement reliées à des capteurs de pression.

Le document WO 2004/020590 décrit un bioréacteur destiné à la culture de cellules vivantes dans lequel les conditions du corps humain sont reproduites artificiellement. Afin d'accéder à une meilleure compréhension de certains dysfonctionnements des mécanismes du corps, il est nécessaire de mettre au point des bioréacteurs capables de mimer in vitro le microenvironnement de tissus anormaux. Une mise en oeuvre proposée par le document décrit une chambre divisée en deux sous-chambres contenant respectivement des cellules d'un premier type et des cellules d'un second type. Les deux sous-chambres sont séparées par une barrière poreuse qui peut être totalement imperméable ou bien perméable à certaines cellules spécifiques. Le bioréacteur comprend également des accès entrée/sortie permettant la circulation de cellules, fluides ou produits chimiques dans chacune des sous-chambres. Par l'ajout de différents substrats positionnés de manière adaptée dans le bioréacteur, il est possible de procéder à des mesures électrochimiques et optiques.

Ainsi, une grande variété de dispositifs de filtration in vitro ont été proposés par le passé.

PRESENTATION DE L'INVENTION

L'invention propose une filtration d'un type nouveau présentant de nombreux avantages par rapport aux solutions proposées dans l'art antérieur. A cet effet, l'invention propose selon un premier aspect un dispositif de filtration caractérisé en ce qu'il comprend :

- un premier bloc présentant une cavité formant une première chambre comportant une paroi inférieure (présentant un ensemble de microstructures comprenant des micromurs et des microplots, l'ensemble de microstructures définissant, sur la paroi inférieure de la chambre de culture, des microchambres et des microcanaux,
- un deuxième bloc présentant une cavité formant une deuxième chambre, et
- une membrane de filtration,
  le premier bloc, la membrane et le deuxième bloc étant agencés de sorte que la membrane soit située entre la première chambre et la deuxième chambre, adjacente à chacune des première et deuxième chambres,
- une première ouverture et une deuxième ouverture pour permettre le passage d'un fluide dans la deuxième chambre séparée de la première chambre par la membrane

Le dispositif de filtration selon le premier aspect de l'invention est avantageusement complété par les caractéristiques suivantes, prises seules ou en une quelconque de leurs combinaisons techniquement possibles :

- une entrée de fluide dans la première chambre connectée à au moins une partie des microcanaux et une sortie de fluide dans la première chambre connectée à au moins une partie des microcanaux sont comprises, les microcanaux formant un réseau reliant l'entrée de fluide à chaque microchambre et chaque microchambre à la sortie de fluide, de sorte à permettre une circulation de fluide dans les microchambres de la première chambre,
- les microchambres présentent une dimension de longueur et une dimension de largeur par rapport à une direction de circulation du fluide chacune comprise entre $500\mu$m et $550\mu$m,
- les microcanaux présentent une dimension de longueur par rapport à une direction de circulation du fluide comprise entre $700\mu$m et $750\mu$m et une dimension de largeur par rapport à une direction de circulation du fluide comprise entre $200\mu$m et $250\mu$m,
- les microchambres comprennent une zone d'entrée et une zone de sortie, les micromurs comprenant des zones anguleuses de part et d'autre de la zone d'entrée et de la zone de sortie d'au moins une chambre, les zones anguleuses présentant une dimension de largeur par rapport à une direction de circulation du fluide comprise entre $100\mu$m et $120\mu$m de sorte à définir, de part et d'autre de la zone d'entrée de ladite chambre des zones partiellement protégées,
- la première chambre présente une surface de culture dans un rapport avec une surface globale de la paroi inférieure compris entre 90% et 110%,
- la première chambre présente une surface de culture dans un rapport avec un volume global disponible de fluide de la première chambre compris entre 4/mm et 6/mm,
- la deuxième chambre comprend une paroi supérieure présentant un ensemble de microstructures identique à celui de la paroi inférieure de la première chambre,
- la membrane est en matériau souple,
- la membrane est en matériau hydrophile,
- la membrane est en matériau hydrophobe,
- la membrane est une membrane barrière,
- la membrane est en matériau adapté pour permettre une culture de cellules sur la membrane, et
- un moyen de maintien présentant une configuration verrouillée dans laquelle sont maintenus solidaires d'une part le premier bloc et la membrane, d'autre part la membrane et le deuxième bloc, et une configuration déverrouillée, dans laquelle le bloc et la membrane sont séparables l'un de l'autre et/ou dans laquelle la membrane et le deuxième bloc sont séparables l'une de l'autre, le moyen de maintien pouvant être basculé de la configuration verrouillée vers la configuration déverrouillée et inversement, est compris.

L'invention propose également, selon un deuxième aspect, un système de filtration, caractérisé en ce qu'il comprend

- un dispositif de filtration selon le premier aspect de l'invention, et

- un circuit de fluide comprenant une tuyauterie de circulation munie d'un moyen de circulation et connectée aux première et deuxième ouverture dans la deuxième chambre.

Le système de filtration selon le deuxième aspect de l'invention est avantageusement complété par les caractéristiques suivantes, prises seules ou en une quelconque de leurs combinaisons techniquement possibles :

- une entrée de fluide dans la première chambre connectée à au moins une partie des microcanaux et une sortie de fluide dans la première chambre connectée à au moins une partie des microcanaux sont comprises, les microcanaux formant un réseau reliant l'entrée de fluide à chaque microchambre et chaque microchambre à la sortie de fluide, de sorte à permettre une circulation de fluide dans les microchambres de la première chambre, le dispositif comprenant en outre un circuit de fluide comprenant une tuyauterie de circulation munie d'un moyen de circulation et connectée aux entrée et sortie de fluide dans la première chambre,
- le premier circuit comprend un moyen de contrôle pour contrôler une pression de fluide dans la première chambre, et
- le deuxième circuit comprend un moyen de contrôle pour contrôler une pression de fluide dans la deuxième chambre.

Selon un troisième aspect, l'invention propose un système de filtration comprenant plusieurs dispositifs de filtration selon le premier aspect de l'intention connectés par des circuits de circulation.

PRESENTATION DES FIGURES

D'autres caractéristiques, buts et avantages de l'invention ressortiront de la description qui suit, qui est purement illustrative et non limitative, et qui doit être lue en regard des dessins annexés sur lesquels :

- la figure 1 représente, de manière schématique, un dispositif de filtration en vue de face en coupe selon une réalisation possible du premier aspect de l'invention,
- la figure 2 représente, de manière schématique, un dispositif de filtration en vue de côté en coupe selon une réalisation possible du premier aspect de l'invention,
- la figure 3 représente une vue en perspective tridimensionnelle de microstructures selon une réalisation possible du premier aspect de l'invention,
- la figure 4 représente, de manière schématique un système de filtration en coupe en vue de côté selon une réalisation possible du deuxième aspect de l'invention,
- la figure 5 représente un dispositif de filtration en vue de côté en coupe selon une réalisation possible du premier aspect de l'invention dans laquelle la première chambre est pourvue d'une entrée et d'une sortie de fluide,
- la figure 6 représente de manière schématique un dispositif de filtration en vue de haut en coupe selon une réalisation possible du premier aspect de l'invention dans laquelle la première chambre est pourvue d'un réseau d'entrée et d'un réseau de sortie de fluide,
- la figure 7 représente, de manière schématique en vue de haut, des microstructures de la paroi inférieure de la première chambre, ainsi que des dimensions de ces microstructures selon une réalisation possible du premier aspect de l'invention,
- les figures 8a et 8b représentent des images par microscopie électronique de membranes de porosité différentes selon des réalisations possibles du premier aspect de l'invention,
- les figures 9 et 10 représentent, de manière schématique, un système de filtration en vue de côté en coupe selon des réalisations possibles du deuxième aspect de l'invention,
- les figures 11 et 12 représentent graphiquement une évolution du rapport concentration sur concentration initiale dans la deuxième chambre au cours d'une expérience utilisant un système selon une réalisation possible du deuxième aspect de l'invention,
- la figure 13 représente, de manière schématique, un système de filtration en vue de côté en coupe selon des réalisations possibles du deuxième aspect de l'invention, et
- la figure 14 représente, de manière schématique, une expérience pour caractériser la pente à l'eau d'une membrane, laquelle expérience met en oeuvre un système selon une réalisation possible du deuxième aspect de l'invention.

Sur les différentes figures, les éléments similaires portent les mêmes références numériques.

DESCRIPTION DETAILLEE

*Présentation générale*

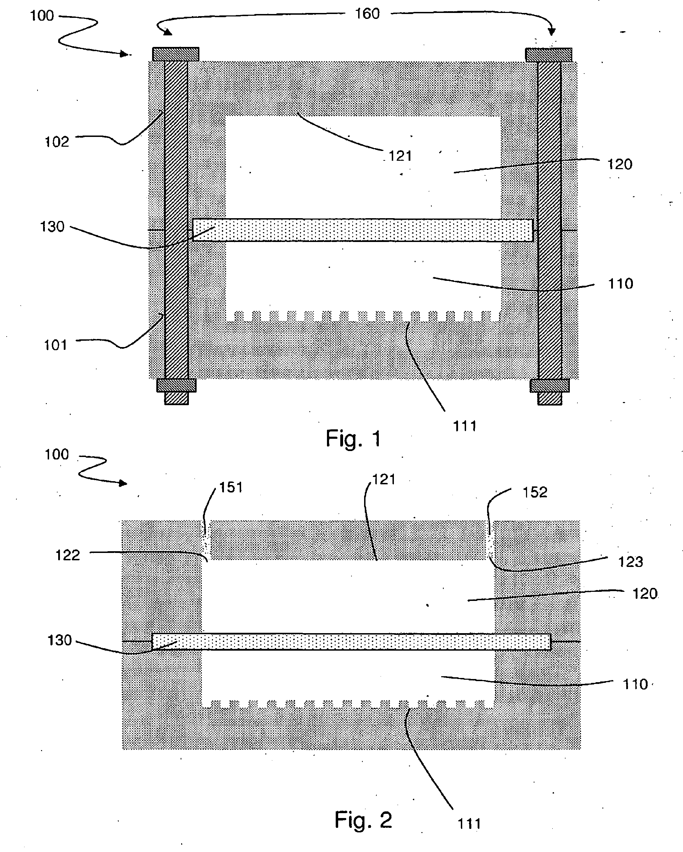

En référence aux figures 1 et 2, un dispositif 100 de filtration selon le premier aspect de l'invention comprend un premier bloc 101 présentant une cavité qui constitue une première chambre 110, un deuxième bloc 102 présentant également une cavité qui constitue une deuxième chambre 102, ainsi qu'une membrane 130 de filtration positionnée entre la première chambre 110 et la deuxième chambre 120 et adjacente à chacune des première et seconde chambres 110, 120.

Ces deux chambres peuvent recevoir un fluide à filtrer ou un fluide de service. Comme on le décrira plus loin, la membrane 130 permet un transport de matière d'un fluide à l'autre par différence dé concentration ou bien par différence de pression entre la première et la deuxième chambre, ou encore par tout autre cause de filtration connue de l'homme de l'art.

A titre d'exemple non limitatif, les premier et deuxième bloc 101, 102 peuvent être constitués de verre, de silice ou avantageusement de polymères tel que le polyméthacrylate de méthyle (PMMA) ou le polydiméthylsiloxane (PDMS), ou un mélange de ceux-ci. Le PDMS présente l'avantage d'être poreux à l'oxygène. Les deux blocs peuvent être constitués du même matériau, ou bien dans des matériaux différents.

La deuxième chambre 120 est destinée à recevoir un fluide en circulation. A cet effet, le dispositif 100 comprend une première ouverture 122 et une deuxième ouverture 123 pour permettre le passage d'un fluide. Elle présente en outre une paroi supérieure 121.

On entend par « membrane » une paroi séparant deux milieux. Une membrane a une porosité dépendant de la taille de ses pores. En l'occurrence, selon le premier aspect de l'invention, la membrane sépare la première chambre 110 et la deuxième chambre 120 et définit un unique espace clos en complémentarité avec chacune de ces chambres 110, 120. En particulier, en l'absence de différence de pression entre les deux chambres 110, 120, la membrane n'a aucun point de contact avec la paroi inférieure 111 de la première chambre 110, ni avec la paroi supérieure 121 de la deuxième chambre 120.

Comme on le décrira en détails plus loin, la première ouverture 122 (respectivement la deuxième ouverture 123) peut être une entrée de fluide dans la deuxième chambre 120 (respectivement une sortie de fluide de la deuxième chambre 120) ou bien une sortie de fluide de la deuxième chambre 120 (respectivement une entrée de fluide dans la deuxième chambre 120) en fonction du sens du courant prévu dans la chambre 120.

La première chambre 110 présente une paroi inférieure 111 ayant préférentiellement une forme sensiblement rectangulaire, ce qui définit une longueur et une largeur.

A titre d'exemple non limitatif, la longueur et la largeur de la paroi inférieure peuvent mesurer respectivement 13.25mm et 11.23mm

Dans la suite, on définit l'horizontale comme parallèle à la paroi inférieure 111 et la verticale comme orthogonale à la paroi 111 et dirigée de la paroi 111 vers la membrane 130. Ainsi, on dira que la paroi inférieure 111 est horizontale et située en dessous de la membrane 130, elle-même située en dessous de la paroi supérieure 121. Par ailleurs, on note d1 direction de longueur de la paroi inférieure 111.

Ces définitions ont pour but de clarifier la suite du texte et ne doivent en aucun cas être interprétées comme une limitation de la position d'utilisation du dispositif selon le premier aspect de l'invention dans un repère quelconque.

*Microstructures pour cultiver des cellules*

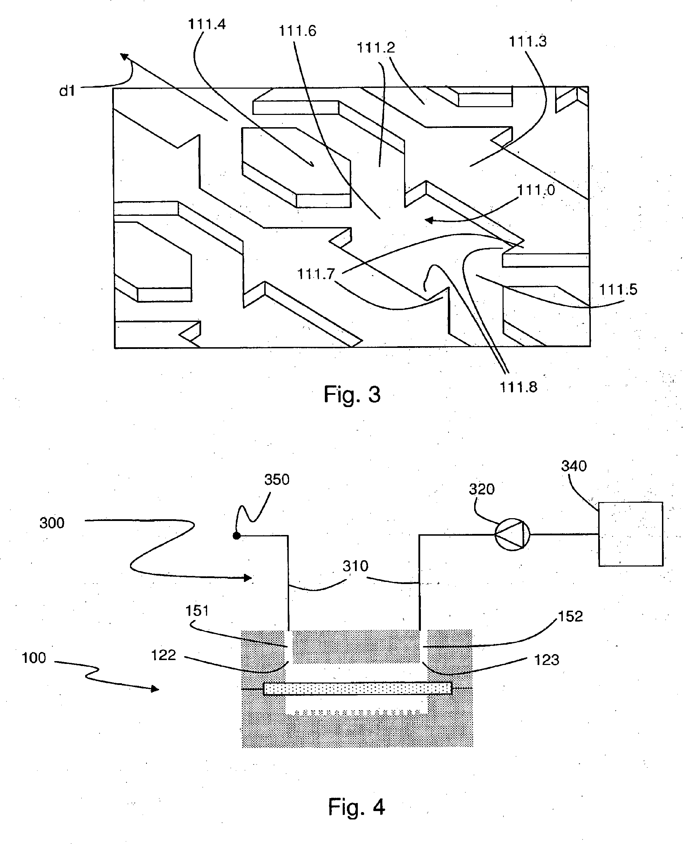

La première chambre 110 est destinée à recevoir une culture de cellule. A cet effet, la paroi inférieure 111 présente un ensemble de microstructures représentées en trois-dimensions sur la figure 3. Ces microstructures ont été développées par la Demanderesse et ont déjà fait l'objet, dans leur forme, d'un rapport de thèse : « Développement et caractérisation d'une puce à cellules pour le criblage d'agents toxiques ».

Par ailleurs, la Demanderesse a déposé le 23 juin 2009 une demande de brevet FR 0954288 revendiquant des dimensionnements avantageux de ces microstructures, en vue, en particulier, d'une application à la culture de cellules hépatiques.

L'ensemble de microstructures comprend :

- des plots 111.4 de dimensions micrométriques désignés par « microplots » dans la suite, et
- des murs 111.3 de dimensions micrométriques désignés par « micromurs » dans la suite.

On entend par « surface globale » de la paroi inférieure 111 la surface qu'aurait la paroi inférieure 111 si on projetait les microstructures sur la paroi 111 dans la direction verticale. Il s'agit de la surface du rectangle que forme la paroi 111.

Les micromurs 111.3 présentent des parties en forme de flèche et des parties droites, et s'étendent sur toute la longueur de la paroi inférieure 111.

Un micromur 111.3 définit des microchambres 111.0 au niveau de ses parties en forme de flèche en complémentarité avec un autre micromur 111.3, et des microcanaux 111.2 au niveau de ses parties droites en complémentarité avec des microplots 111.4, comme représenté sur la figure 2.

Chaque microplot 111.4 définit deux microcanaux situés de part et d'autre du microplot, chacun en complémentarité avec un micromur.

La paroi inférieure 111 de la première chambre 110 comprend ainsi des lignes périodiques dans sa direction d1 de longueur, sur toute sa largeur. Chaque ligne comprend une alternance de microchambres 111.0 et de microcanaux 111.2, deux lignes étant séparées par un micromur 111.3.

A titre d'exemple non limitatif, chaque ligne peut comprendre 9 microchambres 111.0 et 8 plots 111.4 - correspondant chacun à deux microcanaux 111.2 - en alternance, la paroi inférieure 111 comprenant un total de 15 lignes.

Un tel dispositif présente sur la paroi inférieure 111 une géométrie favorable au développement de cellules, en particulier par rapport aux dispositifs de culture plans comme les boites de Pétri. Les microstructures permettent une organisation des cellules en trois dimensions, à condition de les alimenter en fluide nutritif contenant des éléments nécessaires au développement des cellules, en particulier de l'oxygène ou du glucose.

*Culture de cellules en fluide stagnant*

A cet effet, le dispositif selon le premier aspect de l'invention permet une alimentation des cellules sans qu'une circulation de fluide nutritif soit nécessaire dans la première chambre. En effet, en disposant un fluide stagnant dans la première chambre où les cellules sont amenées à se développer, et en faisant circuler un fluide nutritif dans la deuxième chambre, les éléments nécessaires au développement des cellules (en particulier le glucose) passent dans le fluide stagnant, pour peu que la membrane 130 soit choisie de façon appropriée. En particulier, on peut choisir une membrane dont la porosité est adaptée pour laisser passer le glucose.

Ainsi, le dispositif 100 selon le premier aspect de l'invention trouve une première application dans la culture de cellules. En effet, un fluide nutritif en circulation peut arracher des cellules et désagréger les structures ce qui constitue un obstacle au développement de cellules et limite leur activité. Le dispositif 100 décrit ci-dessus permet d'outrepasser cette difficulté en proposant une solution à l'alimentation sans circulation de fluide en contact direct avec les cellules.

Toutefois, la pression de fluide en circulation dans la deuxième chambre peut entrainer une déformation de la membrane 130 qui se rapproche alors de la paroi inférieure 111. La membrane 130 constitue alors une menace mécanique pour les cellules en développement ; elle risque de désagréger les structures de cellules et de les arracher de la paroi 111.

Or, les microstructures protègent les cellules de cet effet néfaste. En effet, même si la membrane 130 venait au contact de la paroi inférieure 111, elle serait au contact des micromurs 111.3 et des microplots 111.4, les cellules pouvant toujours se développer dans les microchambres 111.0 et les microcanaux 111.2. Ainsi, les microchambres et les microcanaux constituent une structure non seulement favorable au développement des cellules, mais aussi protégée dans le cas où la membrane 130 se déforme jusqu'à la paroi inférieure 111.

Le dispositif 100 ainsi décrit peut être utilisé dans un système de filtration selon le deuxième aspect de l'invention comme on l'a représenté sur la figure 4. Le système comprend, outre un dispositif 100, un circuit 300 de fluide comprenant une tuyauterie de circulation 310 pourvu d'un moyen 320 de circulation. On entend par « tuyauterie » un ensemble d'un ou plusieurs tuyaux. Ces tuyaux peuvent être souples ou rigides, et constitués de tout matériau adapté connu de l'homme de l'art. Le circuit de fluide 300 est relié aux première et deuxième ouvertures 122, 123 dans la deuxième chambre 120, par l'intermédiaire de passages respectifs 151 et 152 dans le deuxième bloc 102.

Le moyen 320 de circulation est préférentiellement relié à une réserve 340 en milieu nutritif, et peut comprendre une pompe liquide, une pompe péristaltique, un jeu de vannes, par exemple des électrovannes, ou tout autre moyen adapté connu de l'homme de l'art.

En outre, le circuit 300 comprend préférentiellement une évacuation 350 pour le fluide nutritif après passage dans la deuxième chambre 120.

Un tel système selon le deuxième aspect de l'invention n'est pas limité à cette représentation dans lequel le circuit 300 est ouvert, et s'étend en particulier à tout système dans lequel le circuit 300 est fermé et comprend optionnellement un moyen de régénération du fluide nutritif.

*Circulation de fluide dans la première chambre*

Par ailleurs, le dispositif 100 selon le premier aspect de l'invention n'est pas limité à la description qui en a été faite jusqu'ici. En variante, on prévoit la possibilité de faire circuler un fluide également dans la première chambre 110.

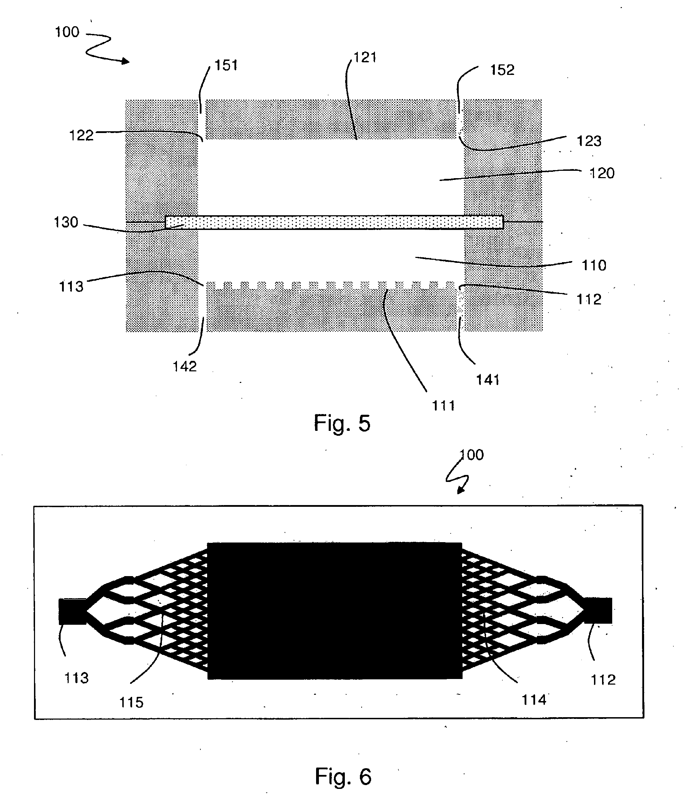

A cet effet, le dispositif 100 selon le premier aspect de l'invention comprend en outre une entrée 112 de fluide et une sortie 113 de fluide comme représenté sur la figure 5.

L'entrée 112 de fluide est connectée à au moins une partie des microcanaux 111.2 par l'intermédiaire d'un réseau 114 d'entrée. Le réseau 114 d'entrée comprend des ramifications successives pour alimenter chacune des lignes de la paroi inférieure 111 de la première chambre à partir de l'entrée 112 de fluide.

Le fluide est destiné à circuler au niveau des microcanaux 111.2 et des microchambres 111.0, et au dessus des microstructures dans la première chambre 110, par exemple pour alimenter les cellules en développement.

La sortie 113 de fluide est connectée à au moins une partie des microcanaux par l'intermédiaire d'un réseau 115 de sortie. Le réseau 115 de sortie comprend des points de confluence successifs pour relier chacune des lignes de la paroi inférieure 111 de la chambre 110 à la sortie 113 de fluide. On a représenté sur la figure 6 les réseaux d'entrée 114 et de sortie 115 de la paroi inférieure dans cette variante avantageuse de l'invention.

Ainsi, les microcanaux 111.2 forment un réseau reliant l'entrée 112 de fluide à chaque microchambre 111.0 - par l'intermédiaire du réseau 114 d'entrée - et chaque microchambre à la sortie 113 de fluide - par l'intermédiaire du réseau 115 de sortie. Les microchambres 111.0 comprennent préférentiellement une zone d'entrée 111.5 et une zone de sortie 111.6 pour permettre la circulation du fluide sensiblement dans la direction d1 - direction de longueur de la paroi 111.

On envisage de nombreuses applications du dispositif selon cette variante avantageuse du premier aspect de l'invention.

Par exemple, on peut prévoir de faire circuler dans la chambre 110 un fluide contenant des molécules à tester sur les cellules. Cette application est particulièrement intéressante dans le dépistage de substances toxiques pour l'être humain : on cultive un tissu cellulaire humain, nourri à travers la membrane par un fluide nutritif circulant dans la chambre 120 et exposé directement à des molécules tests dans la chambre 110.

Par ailleurs, on peut faire circuler dans la chambre 110 un fluide d'évacuation pour éliminer en continu les sécrétions cellulaires.

Une autre application possible est la stimulation mécanique des cellules Certaines cellules, comme les cellules endothéliales, sont naturellement soumises à des conditions découlement, comme le sang. Ces cellules sont naturellement activées par frottement mécanique, ce que l'on peut reproduire par circulation du fluide dans la première chambre 110,

*Dimensions des microstructures*

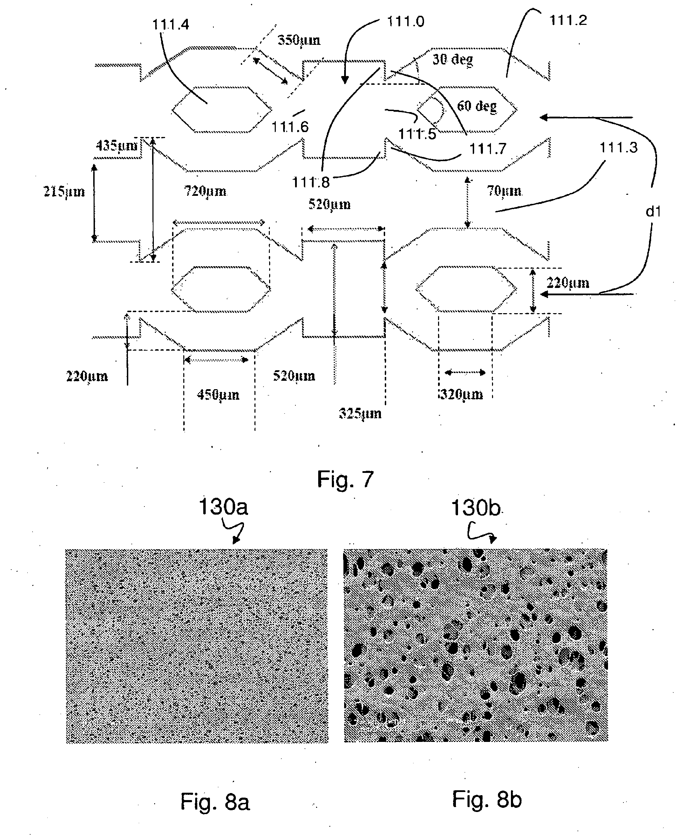

On va maintenant décrire des dimensionnements avantageux des microstructures selon une réalisation possible du premier aspect de l'invention en référence à la figure 7.

Dans cette variante avantageuse, les microchambres 111.0 présentent une dimension de longueur et une dimension de largeur par rapport à la direction d1 chacune comprise entre $500\mu m$ et $550\mu m$, préférentiellement de $520\mu m$. Ainsi, les dimensions des microchambres 111.0 du dispositif de filtration 100 sont avantageusement $520\mu m$ x $520\mu m$ x $100\mu m$.

Ces dimensions sont particulièrement favorables au développement de cellules hépatiques dans les microchambres 111.0.

Avantageusement encore, les microcanaux 111.2 présentent par rapport à la direction d1 une dimension de longueur comprise entre $700\mu m$ et $750\mu m$, préférentiellement de $720\mu m$, et une dimension de largeur comprise entre $200\mu m$ et $250\mu m$, préférentiellement de $220\mu m$. Ainsi, les dimensions des microcanaux 111.2 du dispositif 100 sont avantageusement $720\mu m$ x $220\mu m$ x $100\mu m$.

Les microcanaux 111.2 ainsi dimensionnés facilitent le développement de cellules hépatiques ; ils permettent en particulier la migration de morceau d'organe de foie à travers le réseau de microcanaux.

On a par ailleurs représenté sur la figure 7 plusieurs autres dimensions caractéristiques des micromurs 111.3, des microplots 111.4 et des microcanaux 111.2 pour une réalisation possible du dispositif de filtration 100 du premier aspect de l'invention.

Selon une variante avantageuse, les micromurs 111.3 comprennent des zones anguleuses 111.7 de part et d'autre de la zone d'entrée 111.5 et de la zone de sortie 111.6 d'au moins une chambre 111.0, comme représenté sur les figures 3 et 7.

Ces zones anguleuses 111.7 présentent une dimension de largeur par rapport à la direction d1 avantageusement comprises entre $100\mu m$ et $120\mu m$, préférentiellement de $110\mu m$. En particulier, elles présentent une arête transversale à la direction d1 de circulation du fluide. Les zones anguleuses 111.7 définissent, par rapport à la direction d1, des zones partiellement protégées de part et d'autre de la zone d'entrée 111.5 de ladite chambre 110, c'est-à-dire des zones où la circulation du fluide est brutalement ralentie.

Ainsi, les cellules se développant dans de telles zones partiellement protégées sont peu susceptibles d'être arrachées par le fluide circulant dans la microchambre 111.0.

Naturellement, l'étendue d'une zone où les cellules sont protégées d'un arrachement par le fluide dépend des conditions de circulation, en particulier du débit et du cisaillement

Néanmoins, les zones partiellement protégées présentent, selon cette variante avantageuse du premier aspect de l'invention, une arête transversale à la direction d1 d'une largeur d'au moins 100$\mu$m.

Elles permettent ainsi une agrégation des cellules au niveau de coins 111.8 de la microchambre 111.0 disposés transversalement de part et d'autre et en aval de la zone d'entrée 111.5 par rapport à la direction d1. En particulier, des cellules hépatiques sont susceptibles de s'agréger en sphéroïde de diamètre important, de l'ordre de 100$\mu$m, forme favorable à une bonne activité cellulaire.

De manière générale, le réseau de microcanaux permet aux cellules de se développer et de s'agréger en structures tridimensionnelles au niveau de zones de développement, à savoir :

- la partie de la paroi inférieure 111 au niveau des microchambres 111.0,
- la partie de la paroi inférieure 111 au niveau des microcanaux 111.2,
- les parois latérales des micromurs 111.3, et
- les parois latérales des microplots 111.4.

On désigne par « surface de culture » l'ensemble de ces zones de développement. Avantageusement, le rapport entre la surface de culture et la surface globale de la paroi inférieure 111 est compris entre 90% et 110%. Il est préférentiellement égal à 100% à une précision d'1% près.

A titre d'exemple numérique non limitatif, la surface de culture peut être décomposée de la manière suivante (pour une surface globale de la paroi inférieure de 149mm$^2$) :

- surface de la paroi inférieure au niveau des microchambres 111.0 et des microcanaux 111.2 : 96.5 mm$^2$,
- surface des parois latérales des micromurs 111.3 : 36.5mm$^2$
- surface des parois latérales des microplots 111.4 : 18mm$^2$,

La surface de culture est donc de 151mm$^2$.

Le rapport de surface de culture sur la surface globale de la paroi inférieure est donc, dans cet exemple, de 101%.

Ainsi, les microstructures sur la paroi inférieure 111 ne modifient presque pas la surface disponible pour la culture par rapport à la surface globale de la paroi inférieure 111, tout en permettant un développement tridimensionnel.

En outre, des cellules peuvent aussi se développer sur les surfaces supérieures des micromurs 111.3 et des microplots 111.4, bien que de telles zones ne soient pas particulièrement favorables au développement tridimensionnel.

On appelle alors « surface de culture totale » la surface de culture définie précédemment à laquelle on ajoute les surfaces supérieures des micromurs 111.3 et des microplots 111.4.

Dans l'exemple numérique précédent, ces surfaces supérieures sont de 52.5mm$^2$ et la surface de culture totale est de 203.5mm$^2$ et le rapport entre la surface de culture totale et la surface globale de la paroi inférieure est de 137%.

Avantageusement encore, la chambre 110 de culture présente un volume et le rapport R2 entre la surface de culture totale et le volume de la chambre 110 de culture est compris entre 4mm$^{-1}$ et 6mm$^{-1}$

Si le volume est trop faible par rapport à la surface de culture totale (R2 > 6 mm$^{-1}$) les cellules risquent d'être confinées et de ne pas disposer d'assez de nutriments distribués par le fluide, ce qui est néfaste à leur développement.

Par ailleurs, un volume trop important par rapport à la surface de culture totale (R2 < 4 mm$^{-1}$), n'est pas intéressant ; il est en effet préférable de disposer de dispositifs miniaturisés.

On entend par volume de la première chambre 110 le volume disponible pour le passage du fluide ; on exclut donc le volume occupé par les micromurs 111.3 et les microplots 111.4.

En reprenant les valeurs de l'exemple numérique précédent, et pour une hauteur de chambre de 0.2mm, on peut déterminer le volume de la chambre 110 de culture : 149 x 0.2 - 52.5 x 0.1 = 24.55mm$^3$

Le rapport entre surface de culture totale et volume de la première chambre 110 est alors de 203.5mm$^2$/24.55mm$^3$ = 8.29mm$^{-1}$.

Avantageusement, la surface supérieure 121 de la deuxième chambre 120 présente elle-aussi des microstructures. Ces microstructures peuvent présenter toutes les variantes avantageuses des microstructures détaillées jusqu'ici concernant la paroi inférieure 111 de la première chambre 110. Les microstructures peuvent être de dimensions identiques sur les parois inférieure 111 et supérieure 121, ou bien de dimensions différentes. Cette variante est particulièrement intéressante pour une application en vue d'une culture de cellules dans les deux chambres

*Membranes envisagées dans le cadre de l'invention*

On va maintenant décrire plus en détail différentes caractéristiques de la membrane 130 envisagées dans le cadre de l'invention.

La membrane 130 est de préférence en matériau souple et peut ainsi être légèrement déformable en fonction de la pression régnant dans chacune des chambres 110, 120. Comme on l'a vu précédemment, les microstructures empêchent la membrane 130 de coller aux parois et protègent les cellules de culture d'un éventuel contact avec la membrane 130.

La membrane 130 peut être hydrophile ou hydrophobe, et sera sélectionnée en fonction de l'application visée (dialyse, culture cellulaire, ...)..

Une membrane est dite hydrophile lorsqu'il y a interaction des groupements terminaux avec l'eau par liaison hydrogène. Les membranes hydrophiles, comme les membranes cellulosiques, ont une bonne diffusion et comme elles ont une faible adsorption des protéines, elles ont une bonne convection; par contre la biocompatibilité est médiocre. Elles sont utilisées en dialyse pour laisser passer l'eau et les très petits solutés.

Les membranes hydrophobes, comme les synthétiques, ont une diffusion plus faible, mais un coefficient d'ultrafiltration plus élevé du fait de leur structure poreuse qui contrebalance l'effet négatif de l'adsorption des protéines; ce dernier est à l'origine de la meilleure biocompatibilité. Elles sont souvent utilisées en hémodiafiltration. La biocompatibilité des membranes entrain de nombreuses applications de ces membranes avec la culture de cellules.

Les membranes peuvent être microstructurées avec des microstructures de type micro pores (voir figures 8a et 8b) mais aussi avec des micro pores qui pourraient aussi prendre la forme de microcanaux ou micro pilliers ou micro géometries, par exemple des géométries telles qu'illustrées sur la figure 7.

Avantageusement, la membrane 130 peut être hydrophile, ce qui limite l'adhésion de bactéries et de protéines et diminue la résistance du passage de fluide dans les pores de la membrane 130.

Alternativement, la membrane 130 peut être hydrophobe, ce qui limite l'adhésion de bactéries, facilite l'adhésion de protéines et augmente la résistance du passage du fluide dans les pores.

Différentes porosités peuvent être utilisées pour la membrane 130 en fonction de la taille des éléments à filtrer. Selon une variante avantageuse, la membrane 130 est de préférence une membrane barrière, c'est-à-dire qu'elle ne laisse diffuser que les petites molécules ou les gaz et empêche le passage de fluides d'une chambre 110, 120, vers l'autre chambre 120, 110. Les figures 8a et 8b montrent des images prises par microscope électronique de deux exemples de membranes 130a, 130b de filtration en polyethersulfone d'une porosité respective de 40000 Da et 500000 Da.

Selon une réalisation possible de l'invention, la membrane 130 est choisie pour permettre la culture de cellules sur la membrane 130. En particulier, on prévoit de cultiver avantageusement des cellules modèles des barrières biologiques (par exemple barrière intestinale ou barrière encéphalique) sur la membrane, comme des cellules MDCK ou Caco-2.

Préférentiellement, mais non limitativement, la membrane 130 présente une surface de l'ordre du cm$^2$.

*Séparation des blocs et de la membrane*

Avantageusement, le dispositif 100 de filtration selon le premier aspect de l'invention comprend un moyen 160 de maintien pour maintenir ensemble le premier bloc 101, la membrane 130 et le deuxième bloc 102 dans cet ordre.

Le moyen 160 présente une configuration verrouillée dans laquelle sont maintenus solidaires d'une part le premier bloc 101 et la membrane 130, d'autre part la membrane 130 et le deuxième bloc, et une configuration déverrouillée, dans laquelle le bloc 101 et la membrane 130 sont séparables l'un de l'autre et/ou dans laquelle la membrane 130 et le deuxième bloc 102 sont séparables l'une de l'autre.

En outre, le moyen 160 de maintien peut être basculé de la configuration verrouillée vers la configuration déverrouillée et inversement, par exemple par action d'un utilisateur.

Le moyen 160 peut comprendre des vis traversant sur toute leur hauteur le premier bloc 101, la membrane 130 et le deuxième bloc 102, comme on l'a représenté sur la figure 1. Le moyen 160 peut aussi comprendre des taquets, un étau, ou encore tout autre moyen adapté connu de l'homme de l'art.

Un tel moyen 160 de maintien présente plusieurs avantages. Il permet de séparer le dispositif 100 en ses constituants et de pouvoir le reconstruire ensuite. Ainsi, on peut accéder aux chambres 110 et 120, ainsi qu'à la membrane 130, sans rendre le dispositif 100 inutilisable.

Ceci est tout particulièrement utile pour nettoyer les chambres des molécules à filtrer qui se sont adsorbées sur les surfaces et les stériliser, par exemple par autoclave ou par tout autre procédé de nettoyage adapté connu de l'homme de l'art.

Par ailleurs, on peut récupérer la membrane 130 pour la soumettre à des analyses, comme des mesures de résistance électrique transmembranaire, la récupération de la membrane pour réaliser des marquages fluorescents sur les cellules, des analyses d'impédance, ou toute autre analyse utile connue de l'homme de l'art. Dans le cas d'une membrane recevant une culture de cellules, le moyen 160 offre aussi la possibilité d'accéder directement à la culture de cellules en évitant l'évacuation de cette culture du dispositif 100 par un fluide qui détruirait la structure de culture.

En outre, la membrane peut être remplacée par une nouvelle membrane pour des expériences répétitives sans nécessité de nettoyage. On peut ainsi réitérer une expérience en changeant la membrane 130 tout en gardant intact le contenu cellulaire des chambres 110, 120.

Inversement, une même membrane 130 peut être transplantée d'un dispositif 100 à un autre et faire l'objet d'expériences avec des chambres 110, 120 à géométrie de microstructures différentes. Ceci peut être utile pour caractériser l'effet des microstructures sur la filtration ou sur une culture de cellules sur la membrane 130..

*Système de filtration à deux circuits de fluide*

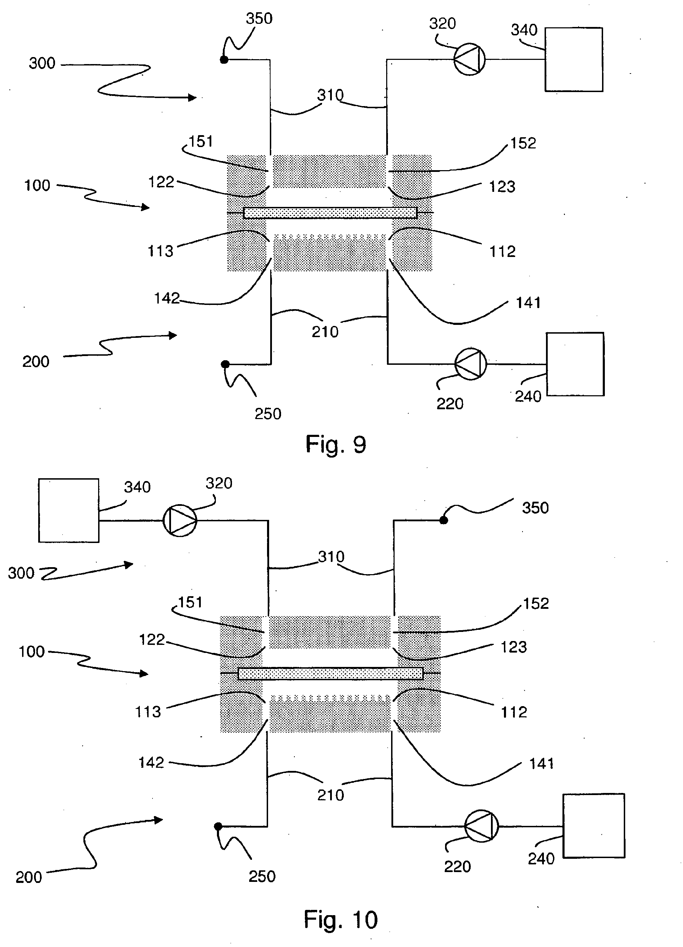

On va maintenant décrire, en regard des figures 9 à 10, un système de filtration selon plusieurs réalisations possibles du deuxième aspect de l'invention.

Dans les variantes envisagées ci-dessous, le dispositif 100 intégré dans le système de filtration comprend une entrée 112 de fluide et une sortie 113 de fluide dans la première chambre 110.

Le système comprend un circuit 200 de fluide connecté à la première chambre 110, analogue au circuit 300 connecté à la deuxième chambre 120 qu'on a déjà décrit plus haut. Le circuit 200 de fluide comprend une tuyauterie de circulation 210 munie d'un moyen 220 de circulation et est connectée aux entrées 112 et sortie 113 dans la première chambre 110. Le circuit de fluide 200 est relié aux entrée 112 et sortie 113 de fluide dans la première chambre 110, par l'intermédiaire de passages respectifs 141 et 142 dans le premier bloc 101.

Le moyen 220 de circulation est préférentiellement relié à une réserve 240 en fluide, et peut comprendre une pompe liquide, une pompe péristaltique, un jeu de vannes, par exemple des électrovannes, ou tout autre moyen adapté connu de l'homme de l'art.

En outre, le circuit 200 comprend préférentiellement une évacuation 250 pour le fluide après passage dans la première chambre 110.

Un tel système selon le deuxième aspect de l'invention n'est pas limité à cette représentation dans lequel le circuit 200 est ouvert, et s'étend en particulier à tout système dans lequel le circuit 200 est fermé et comprend optionnellement un moyen de régénération du fluide.

Par ailleurs, les fluides circulant dans les circuits 200 et/ou 300 sont avantageusement régulés en température. Par exemple, les réserves 240 et/ou 340 peuvent être disposées dans des bains thermostatés (non représentés). Alternativement, ou cumulativement, tout autre moyen de contrôle de la température des circuits 200 et/ou 300 connu de l'homme de l'art peut être envisagé.

Dans la variante de réalisation représentée sur la figure 9, les fluides contenus dans les chambres inférieure 110 et supérieure 120 circulent à co-courant. Selon cette configuration, la première ouverture 122 est une sortie de fluide de la deuxième chambre 120 et la deuxième ouverture 123 est une entrée de fluide dans la deuxième chambre 120.

On a par ailleurs représenté sur la figure 10 une variante de réalisation dans laquelle les fluides contenus dans les chambres inférieure 110 et supérieure 120 circulent à contre-courant. Selon cette configuration, la première ouverture 122 est une entrée de fluide dans la deuxième chambre 120 et la deuxième ouverture 123 est une sortie de fluide de la deuxième chambre 120.

Les systèmes décrits par les figures 9 et 10 trouvent notamment leur application dans le domaine de l'hémodialyse. Du sang à traiter peut circuler dans la deuxième chambre 120 et être débarrassé de certains composants ordinairement éliminés par un rein fonctionnel par filtration à travers la membrane 130. Un fluide de dialyse contenu dans la première chambre 110 permet alors d'évacuer les éléments filtrés et d'assurer un apport en glucose pour le patient.

De tels systèmes peuvent aussi être utilisés pour caractériser une membrane 130 de filtration. On peut par exemple évaluer un coefficient de diffusion d'une molécule pour une membrane 130 donnée à partir d'un modèle physique et de mesures de concentrations de la molécule dans la première chambre 110 et/ou la deuxième chambre 120 en fonction du temps. Le système à co-courant peut être utilisé pour calibrer le modèle et estimer le coefficient de diffusion, et le système à contre-courant peut être utilisé pour vérifier le coefficient de diffusion, ou l'inverse.

En particulier, la Demanderesse a modélisé l'évolution de la concentration au cours du temps dans la première chambre 110, dans le cas où un fluide à filtrer circule dans la deuxième chambre 120, par les équations suivantes :

- dans le cas d'un écoulement à co-courant (comme sur la figure 9) :

$$C_1(t) = C_{1\infty} + \left(C_{1o} - C_{\infty}\right)\exp\left[-\frac{Lb\,D_m}{\delta}\left(\frac{V_1 + V_2}{V_1 V_2}\right)t\right]$$

- dans le cas d'un écoulement à contre-courant (comme sur la figure 10) :

$$C_1(t) = C_{1\infty} + (C_{1o} - C_\infty)\exp\left[-Q_f\left(1 + \frac{2}{Pe}\right)\left(\frac{V_1 + V_2}{V_1 V_2}\right)t\right],$$

où $Pe = \dfrac{Q_f}{(bL/2)}\dfrac{\delta}{D_m}$ , et

$C_1(t)$ représente la concentration dans la première chambre 110 en fonction du temps t,

L représente la longueur (commune) des première et deuxième chambres 110, 120,

b représente la largeur (commune) des première et deuxième chambres 110, 120, $Q_f$ représente le débit de filtration,

$V_1$ représente le volume de la première chambre 110,

$V_2$ représente le volume de la deuxième chambre 120,

$C_{10}$ représente la concentration initiale dans la première chambre 110,

$C_{1\infty}$ représente la concentration d'équilibre dans la première chambre 110,

$D_m$ représente le coefficient de diffusion de la molécule à filtrer pour la membrane 130, et

$\delta$ représente l'épaisseur de la membrane.

Grace à ces modèles, il est possible de déterminer les valeurs du coefficient de diffusion Dm des molécules testées pour une membrane 130 donnée. Pour cela, on peut par exemple travailler en mode co-courant et mesurer la disparition de la molécule dans la deuxième chambre 120 (et donc son apparition dans la première chambre 110).

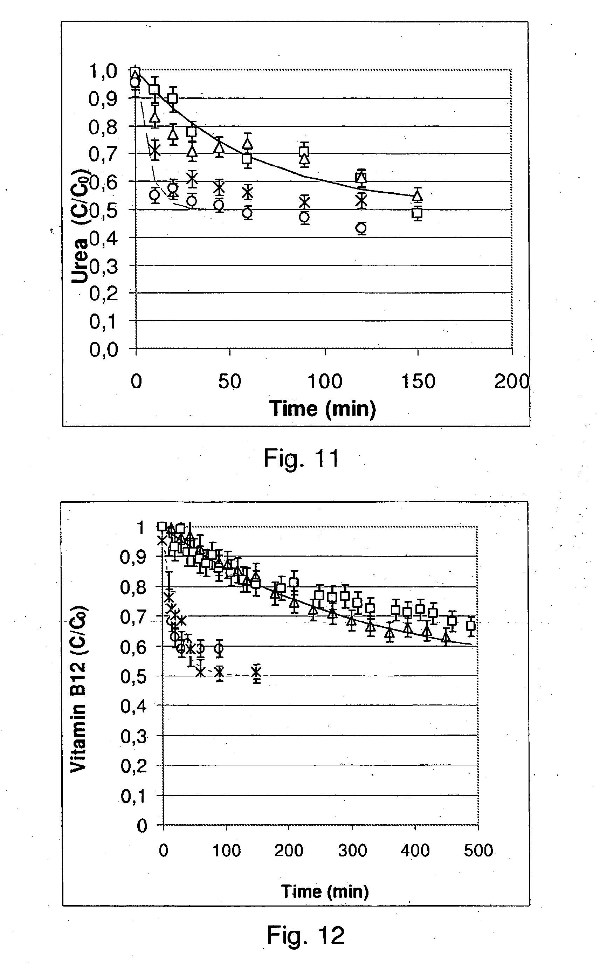

On a représenté sur les figures 11 et 12 l'évolution du rapport concentration sur concentration initiale dans la deuxième chambre 120 en fonction du temps pour des conditions d'expérience donnée, respectivement pour l'urée (figure 11) et la vitamine B12 (figure 12), avec plusieurs types de membrane. Par ailleurs, la Demanderesse a aussi déterminé ce rapport pour l'albumine (graphique non représenté).

Les résultats expérimentaux sont représentés, pour une expérience avec la membrane à forte porosité (de type 8F), par les icones □ ,Δ, et pour une expérience avec membrane à faible porosité (de type 1 FPH), par les icones: *, o. On fait ensuite une régression du modèle analytique, par exemple par une minimisation aux moindres carrés, ce qui permet d'obtenir expérimentalement les coefficients de diffusion répertoriés (en $m^2/s$) dans le tableau suivant. :

|  | Urée | Vitamine B12 | Albumine |
|---|---|---|---|
| 1FPH | $2 \times 10^{-12} m^2/s$ | $4 \times 10^{-13} m^2/s$ | $9 \times 10^{-14}\ m^2/s$ |
| 8F | $2 \times 10^{-11}\ m^2/s$ | $6 \times 10^{-12}\ m^2/s$ | $2 \times 10^{-13}\ m^2/s$ |

Ainsi plus la molécule est grosse plus le coefficient de diffusion est petit. En outre, plus la membrane est poreuse plus le coefficient de diffusion est important.

La validité du coefficient de diffusion a ensuite été prouvée à l'aide du modèle à contre-courant.

Ainsi, on peut caractériser une membrane avec un microsystème nécessitant une surface de membrane de l'ordre du $cm^2$. Si nécessaire, les caractéristiques estimées peuvent être extrapolées sur des membranes de taille supérieure.

*Régulation de pression dans les chambres*

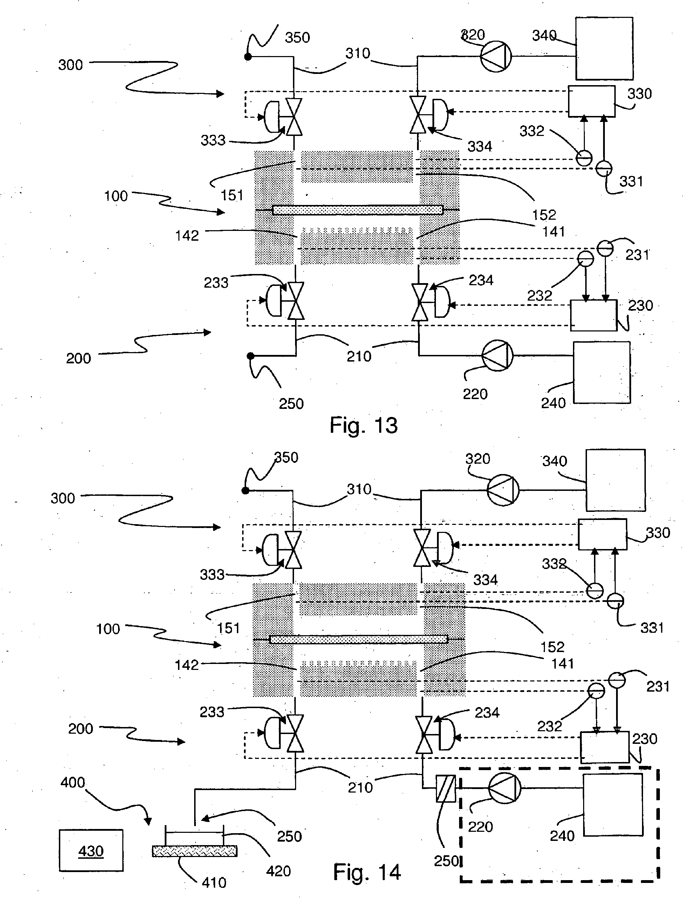

En référence à la figure 13, le circuit 200 de circulation comprend avantageusement un moyen 230 de contrôle de pression fluidique dans la première chambre 110, ou bien le circuit 300 de circulation comprend avantageusement un moyen 330 de contrôle de pression fluidique dans la deuxième chambre 120, ou bien les deux circuits 200, 300 comprennent chacun un moyen 230, 330 de contrôle de pression dans leur chambre associée 110, 120.

Préférentiellement, mais non limitativement, le moyen 230 de contrôle (respectivement 330) comprend des capteurs de pression 231, 232 (respectivement 331, 332) pour détecter une pression du fluide dans les passages 141, 142 dans le premier bloc 101 (respectivement 151, 152 dans le deuxième bloc 102) ou au niveau des entrée et sortie de fluide 112, 113 (respectivement au niveau des première et deuxième ouvertures 122, 123). Le moyen 230 (respectivement 330) comprend en outre des actionneurs 233, 234 (respectivement 333, 334) disposés sur la tuyauterie 210 (respectivement 310) pour modifier la pression en entrée et en sortie du fluide dans la première chambre 110 (respectivement dans la deuxième chambre 120). Ces actionneurs peuvent être par exemple des électrovannes commandées par une unité de traitement (non représentée) de données en provenance des capteurs de pression, ou tout autre moyen adapté connu de l'homme dé l'art.

Les contrôles de pression peuvent être effectués par l'unité de traitement de chaque moyen de contrôle 230, 330 en stabilisant la pression autour d'une valeur de consigne fixe ou variable par exemple par application d'un régulateur proportionnel, un régulateur proportionnel intégral, une boucle ouverte (auquel cas les capteurs de pression ne sont pas utilisés), ou tout autre boucle de contrôle adaptée connue par l'homme du métier de contrôle de systèmes.

Les moyens de contrôle 230, 330 des circuits 200, 300 peuvent être similaires ou différents selon les besoins de l'application envisagée pour le système de filtration selon le deuxième aspect de l'invention.

Ils s'avèrent très utiles pour la surveillance de certains paramètres au cours de la filtration comme le taux de rejet ou le coefficient de diffusion. Les conditions de débits et le flux transmembranaire peuvent ainsi être étroitement contrôlés.

La différence de pression peut être maintenue à une valeur déterminée de telle sorte que la membrane ne vienne pas adhérer sur les parois.

Par ailleurs, les moyens 230, 330 permettent de contrôler les conditions de débits dans les chambres 110, 120 et le flux transmembranaire lors de la filtration. Ceci permet de réguler des paramètres de la filtration pour un soluté donné, tels que le taux de rejet - c'est-à-dire le pourcentage de matière dissoute retenue par la membrane - et le coefficient de diffusion du soluté à travers la membrane 130.

Ainsi, les moyens 230, 330 permettent de procéder à des expériences répétitives dans les mêmes conditions de débit et de flux transmembranaire.

Une application particulièrement intéressante de cet effet avantageux est de tester différentes membranes dans des conditions similaires d'expérience, ce qui permet de caractériser les propriétés des membranes, par exemple la pente à l'eau- c'est-à-dire la perméabilité hydraulique des membranes à l'eau pure -, par exemple via l'expérience représentée sur la figure 14.

Dans cette expérience, le fluide circulant dans la deuxième chambre 120 est de l'eau. Il n'y a pas de culture de cellules dans la première chambre 110, première chambre 110 qui est en outre isolée de la réserve 240 et du moyen 220 de circulation par un moyen de court-circuit 250 du premier circuit 200. Alternativement, on peut envisager dans cet exemple que le premier circuit 200 ne comprend pas de réserve 240 ni de moyen 220 de circulation, auquel cas le moyen de court-circuit 250 est non nécessaire. Pour représenter ceci, la réserve 240 et le moyen 220 sont entourés d'un rectangle en traits pointillés sur la figure 14.

En outre, l'évacuation 350 du deuxième circuit 300 est bloquante pour eau (par exemple un robinet fermé). Ainsi, l'eau circulant dans la deuxième chambre par la deuxième ouverture 152 ne peut sortir du dispositif 100 que par le passage 142 du premier bloc 101, lequel passage 142 est relié à l'évacuation 250 du premier circuit 200 par la tuyauterie 210.

Par ailleurs, le système selon cette variante du deuxième aspect de l'invention est associée à un dispositif 400 de mesure de masse d'eau, comprenant un conteneur 410 relié à l'évacuation 250, une balance 420 et une unité de traitement 430 en interaction avec la balane 420 et destinée à déterminer la masse d'eau contenue dans le conteneur 410 en fonction du temps. Par ailleurs, l'unité de traitement reçoit des informations des moyens de contrôle 230, 330 de pression dans les deux circuits 200, 300.

Ainsi, le dispositif 400 est capable d'évaluer la masse d'eau filtrant par la membrane 130 en fonction du temps et de la pression transmembranaire, ce qui permet de déterminer la pente à l'eau de la membrane 130.

L'expérience a été menée par la Demanderesse sur les membranes 130a et 130b représentées respectivement sur les figures 8a et 8b. Les pentes à l'eau déterminées sont de $8ml/(min.bar.cm^3)$ et $80ml/(min.bar.cm^3)$ respectivement.

Une autre application est de mesurer les pertes de charges dans la première chambre 110 (et dans la deuxième chambre 120 si elle reçoit une culture de cellules), lesquelles pertes de charges donnent une indication sur les variations du nombre de cellules de culture dans la chambre.

### Dispositifs connectés en séries

Enfin, il est possible d'envisager la mise en série de plusieurs dispositifs 1.00 de filtration permettant ainsi de reproduire intégralement les étapes de fonctionnement d'un rein, chaque dispositif reproduisant une fonction particulière. Plus généralement, on peut utiliser plusieurs dispositifs 100 en série pour reproduire les interactions entre un fluide et différents tissus cellulaires dans l'organisme.

### Conclusion

L'invention présente de nombreux avantages. Le système selon le deuxième aspect de l'invention permet une filtration contrôlée par rapport à de plus gros systèmes. Les circuits de fluides ne connaissent pas de turbulence et que peu d'effet de bords, que ce soit dans la tuyauterie comme dans les chambres du dispositif selon le premier aspect de l'invention. Les moyens de contrôle de pression et la membrane permettent de maintenir des paramètres uniformes (pression, température composition et concentration des fluides) de sorte que les résultats observés sont facilement extrapolables vers des systèmes à paramètres analogues. Ainsi, l'invention constitue un grand pas vers la reproduction in vitro de phénomènes du corps humain ou animal, ainsi que vers la réalisation d'organes artificiels.

**Revendications**

1. Dispositif (100) de filtration **caractérisé en ce qu'**il comprend :

   - un premier bloc (101) présentant une cavité formant une première chambre (110) comportant une paroi inférieure (111) présentant un ensemble de microstructures comprenant des micromurs (111.3) et des microplots (111.4), l'ensemble de microstructures définissant, sur la paroi inférieure (111) de la chambre de culture, des microchambres (111.0) et des microcanaux (111.2),
   - un deuxième bloc (102) présentant une cavité formant une deuxième chambre (120), et
   - une membrane de filtration (130),
   le premier bloc (101), la membrane (130) et le deuxième bloc (102) étant agencés de sorte que la membrane (130) soit située entre la première chambre (110) et la deuxième chambre (120), adjacente à chacune des première et deuxième chambres (110, 120),
   - une première ouverture (122) et une deuxième ouverture (123) pour permettre le passage d'un fluide dans la deuxième chambre (120) séparée de la première chambre (110) par la membrane (130)

2. Dispositif (100) selon la revendication précédente, comprenant en outre :

   - une entrée (112) de fluide dans la première chambre (110) connectée à au moins une partie des microcanaux (111.2),
   - une sortie (113) de fluide dans la première chambre (110) connectée à au moins une partie des microcanaux (111.2),
   les microcanaux formant un réseau reliant l'entrée (112) de fluide à chaque microchambre (111.0) et chaque microchambre (111.0) à la sortie (113) de fluide, de sorte à permettre une circulation de fluide dans les microchambres (111.0) de la première chambre (110).

3. Dispositif (100) de filtration selon l'une des revendications précédentes, dans lequel les microchambres (111.0) présentent une dimension de longueur et une dimension de largeur par rapport à une direction de circulation du fluide chacune comprise entre $500\mu m$ et $550\mu m$.

4. Dispositif (100) de filtration selon l'une des revendications précédentes, dans lequel les microcanaux (111.2) présentent une dimension de longueur par rapport à une direction de circulation du fluide comprise entre $700\mu m$ et $750\mu m$ et une dimension de largeur par rapport à une direction de circulation du fluide comprise entre $200\mu m$ et $250\mu m$.

5. Dispositif (100) de filtration selon l'une dès revendications précédentes, dans lequel les microchambres (111.0) comprennent une zone d'entrée (111.5) et une zone de sortie (111.6), les micromurs (111.3) comprenant des zones anguleuses (117) de part et d'autre de la zone d'entrée (115) et de la zone de sortie (116) d'au moins une chambre (110), les zones anguleuses (117) présentant une dimension de largeur par rapport à une direction de circulation du fluide comprise entre $100\mu m$ et $120\mu m$ de sorte à définir, de part et d'autre de la zone d'entrée (111.5) de ladite chambre (111.0) des zones partiellement protégées.

6. Dispositif (100) de filtration selon l'une des revendications précédentes, dans lequel la première chambre (110) présente une surface de culture dans un rapport avec une surface globale de la paroi inférieure (111) compris entre 90% et 110%.

7. Dispositif (100) de filtration selon l'une des revendications précédentes, dans lequel la première chambre (110) présente une surface de culture dans un rapport avec un volume global disponible de fluide de la première chambre (110) compris entre 4/mm et 6/mm.

8. Dispositif (100) de filtration selon l'une des revendications précédentes, dans lequel la deuxième chambre (120) comprend une paroi supérieure (121) présentant un ensemble de microstructures identique à celui de la paroi inférieure (111) de la première chambre (110).

9. Dispositif (100) de filtration selon l'une des revendications précédentes, dans lequel la membrane (130) est en matériau adapté pour permettre une culture de cellules sur la membrane (130).

10. Dispositif (100) de filtration selon l'une des revendications précédentes, comprenant en outre un moyen (160) de maintien présentant une configuration verrouillée dans laquelle sont maintenus solidaires d'une part le premier bloc (101) et la membrane (130), d'autre part la membrane (130) et le deuxième bloc, et une configuration déverrouillée, dans laquelle le bloc (101) et la membrane (130) sont séparables l'un de l'autre et/ou dans laquelle la membrane (130) et le deuxième bloc (102) sont séparables l'une de l'autre, le moyen (160) de maintien pouvant être basculé de la configuration verrouillée vers la configuration déverrouillée et inversement.

11. Système de filtration, **caractérisé en ce qu'**il comprend

   - un dispositif (100) de filtration selon l'une des revendications 1 à 10, et
   - un circuit (300) de fluide comprenant une tuyauterie (310) de circulation munie d'un moyen (320) de circulation et connectée aux première et deuxième ouvertures (122, 123) dans la deuxième chambre (120).

12. Système de filtration selon la revendication précédente, dans lequel le dispositif (100) de filtration comprend

   - une entrée (112) de fluide dans la première chambre (110) connectée à au moins une partie des microcanaux (111.2),
   - une sortie (113) de fluide dans la première chambre (110) connectée à au moins une partie des microcanaux (111.2),
   les microcanaux formant un réseau reliant l'entrée (112) de fluide à chaque microchambre (111.0) et chaque microchambre (111.0) à la sortie (113) de fluide, de sorte à permettre une circulation de fluide dans les microchambres (111.0) de la première chambre (110),
   le dispositif comprenant en outre un circuit (200) de fluide comprenant une tuyauterie de circulation (210) munie d'un moyen (220) de circulation et connectée aux entrée (112) et sortie (113) de fluide dans la première chambre (110).

13. Système de filtration selon l'une des revendications 11 à 12, dans lequel le premier circuit (200) comprend en outre un moyen (230) de contrôle pour contrôler une pression de fluide dans la première chambre (110).

14. Système de filtration selon l'une des revendications 11 à 13, dans lequel le deuxième circuit (300) comprend en outre un moyen (330) de contrôle pour contrôler une pression de fluide dans la deuxième chambre (120).

15. Système de filtration **caractérisé en ce qu'**il comprend plusieurs dispositifs (100) de filtration selon l'une des revendications 1 à 10 connectés en série par des circuits de circulation.

**Patentansprüche**

1. Filtriervorrichtung (100), **dadurch gekennzeichnet, dass** sie Folgendes umfasst:

   - einen ersten Block (101), der einen Hohlraum aufweist, der eine erste Kammer (110) bildet, umfassend eine untere Wand (111), die eine Einheit von Mikrostrukturen aufweist, umfassend Mikrowände (111.3) und Mikroplots (111.4); wobei die Einheit von Mikrostrukturen auf der unteren Wand (111) der Kultivierkammer Mikrokammern (111.0) und Mikrokanäle (111.2) umfasst,
   - einen zweiten Block (102), der einen Hohlraum aufweist, der eine zweite Kammer (120) bildet, und
   - eine Filtriermembrane (130),
   wobei der erste Block (101), die Membrane (130) und der zweite Block (102) derart angeordnet sind, dass sich die Membrane (130) zwischen der ersten Kammer (110) und der zweiten Kammer (120), benachbart jeder der ersten und der zweiten Kammer (110, 120), befindet,
   - eine erste Öffnung (122) und eine zweite Öffnung (123), um den Durchfluss eines Fluids in die zweite Kammer (120) zu ermöglichen, die von der ersten Kammer (110) durch die Membrane (130) getrennt ist.

2. Vorrichtung (100) nach dem vorhergehenden Anspruch; umfassend außerdem

- einen Fluideingang (112) in der ersten Kammer (110), verbunden mit mindestens einem Teil der Mikrokanäle (111.2);

- einen Fluidausgang (113) in der erste Kammer (110), verbunden mit mindestens einem Teil der Mikrokanäle (111.2),

wobei die Mikrokanäle ein Netz bilden, das den Fluideingang (112) mit jeder Mikrokammer (111.0) und jede Mikrokammer (111.0) mit dem Fluidausgang (113) verbindet, um eine Fluidzirkulierung in den Mikrokammern (111.0) der ersten Kammer (110) zu ermöglichen.

3. Filtriervorrichtung (100) nach einem der vorhergehenden Ansprüche, wobei die Mikrokammern (111.0) eine Längenabmessung und eine Breitenabmessung mit Bezug auf eine Zirkulierungsrichtung des Fluids aufweisen, die jeweils zwischen 500 $\mu$m und 550 $\mu$m liegt.

4. Filtriervorrichtung (100) nach einem der vorhergehenden Ansprüche, wobei die Mikrokanäle (111.2) eine Längenabmessung mit Bezug auf eine Zirkulierungsrichtung des Fluids aufweisen, die zwischen 700 $\mu$m und 750 $\mu$m liegt, und eine Breitenabmessung mit Bezug auf eine Zirkulierungsrichtung des Fluids, die zwischen 200 $\mu$m und 250 $\mu$m liegt.

5. Filtriervorrichtung (100) nach einem der vorhergehenden Ansprüche, wobei die Mikrokammern (111.0) einen Eingangsbereich (111.5) und einen Ausgangsbereich (111.6) umfassen, wobei die Mikrowände (111.3) Winkelbereiche (117) auf beiden Seiten des Eingangsbereichs (115) und des Ausgangsbereichs (116) mindestens einer Kammer (110) umfassen, wobei die Winkelbereiche (117) eine Breitenabmessung mit Bezug auf eine Zirkulierungsrichtung des Fluids aufweisen, die zwischen 100 $\mu$m und 120 $\mu$m liegt, um auf beiden Seiten des Eingangsbereichs (111.5) der Kammer (111.0) teilweise geschützte Bereiche zu definieren.

6. Filtriervorrichtung (100) nach einem der vorhergehenden Ansprüche, wobei die erste Kammer (110) eine Kultivierfläche in einem Bezug mit einer globalen Fläche der unteren Wand (111) zwischen 90 % und 110 % aufweist.

7. Filtriervorrichtung (100) nach einem der vorhergehenden Ansprüche, wobei die erste Kammer (110) eine Kultivierfläche in einem Bezug mit einem verfügbaren globalen Volumen von Fluid der ersten Kammer (110) zwischen 4/mm und 6/mm aufweist.

8. Filtriervorrichtung (100) nach einem der vorhergehenden Ansprüche, wobei die zweite Kammer (120) eine obere Wand (121) umfasst, die eine Einheit von Mikrostrukturen aufweist, die identisch mit derjenigen der unteren Wand (111) der ersten Kammer (110) ist.

9. Filtriervorrichtung (100) nach einem der vorhergehenden Ansprüche, wobei die Membrane (130) aus einem Material besteht, das ausgelegt ist, um eine Zellkultur auf der Membrane (130) zu ermöglichen.

10. Filtriervorrichtung (100) nach einem der vorhergehenden Ansprüche, umfassend außerdem ein Haltemittel (160), das eine verriegelte Konfiguration aufweist, in der einerseits der erste Block (101) und die Membrane (130) und andererseits die Membrane (130) und der zweite Block fest miteinander verbunden gehalten werden, und eine entriegelte Konfiguration, in der der Block (101) und die Membrane (130) voneinander getrennt werden können, und/oder in der die Membrane (130) und der zweite Block (102) voneinander getrennt werden können, wobei das Haltemittel (160) von der verriegelten Konfiguration in die entriegelte Konfiguration und umgekehrt gekippt werden kann.

11. Filtriersystem, **dadurch gekennzeichnet, dass** es Folgendes umfasst:

- eine Filtriervorrichtung (100) nach einem der Ansprüche 1 bis 10, und
- einen Fluidkreislauf (300), umfassend eine Zirkulierungsrohrleitung (310), die mit einem Zirkulierungsmittel (320) ausgestattet und mit der ersten und der zweiten Öffnung (122, 123) in der zweiten Kammer (120) verbunden ist.

12. Filtriersystem nach dem vorhergehenden Anspruch, wobei die Filtriervorrichtung (100) Folgendes umfasst:

- einen Fluideingang (112) in der ersten Kammer (110), verbunden mit mindestens einem Teil der Mikrokanäle (111.2),
- einen Fluidausgang (113) in der ersten Kammer (110), verbunden mit mindestens einem Teil der Mikrokanäle (111.2),

wobei die Mikrokanäle ein Netz bilden, das den Fluideingang (112) mit jeder Mikrokammer (111.0) und jede Mikrokammer (111.0) mit dem Fluidausgang (113) verbindet, um eine Fluidzirkulierung in den Mikrokammern (111.0) der ersten Kammer (110) zu ermöglichen,

wobei die Vorrichtung außerdem einen Fluidkreislauf (200) umfasst, umfassend eine Zirkulierungsrohrleitung (210), die mit einem Zirkulierungsmittel (220) ausgestattet und mit dem Fluideingang (112) und -ausgang (113) in der ersten Kammer (110) verbunden ist.

13. Filtriersystem nach einem der Ansprüche 11 bis 12, wobei der erste Kreislauf (200) außerdem ein Steuermittel (230) umfasst, um einen Fluiddruck in der ersten Kammer (110) zu steuern.

14. Filtriersystem nach einem der Ansprüche 11 bis 13, wobei der zweite Kreislauf (300) außerdem ein Steuermittel (330) umfasst, um einen Fluiddruck in der zweiten Kammer (120) zu steuern.

15. Filtriersystem, **dadurch gekennzeichnet, dass** es mehrere Filtriervorrichtungen (100) nach einem der Ansprüche 1 bis 10 umfasst, die in Reihe durch Zirkulierungskreisläufe verbunden sind.

**Claims**

1. Filtration device (100) **characterised in that** it comprises:

- a first block (101) having a cavity forming a first chamber (110) comprising a bottom wall (111) having a set of microstructures comprising microwalls (111.3) and micro-contacts (111.4); with the set of microstructures defining, on the bottom wall (111) of the culture chamber, micro-bumps (111.0) and micro-channels (111.2),
- a second block (102) having a cavity forming a second chamber (120), and
- a filtration membrane (130),

with the first block (101), the membrane (130) and the second block (102) being arranged such that the membrane (130) is located between the first chamber (110) and the second chamber (120), adjacent to each of the first and second chambers (110, 120),

- a first opening (122) and a second opening (123) for enabling a first fluid to pass into the second chamber (120) which is separated from the first chamber (110) by the membrane (130)

2. Device (100) as claimed in the preceding claim, further comprising:

- a fluid inlet (112) in the first chamber (110) connected to at least one portion of the micro-channels (111.2),
- a fluid outlet (113) in the first chamber (110) connected to at least one portion of the micro-channels (111.2), with the micro-channels forming a network connecting the fluid inlet (112) to each micro-chamber (111.0) and each micro-chamber (111.0) to the fluid outlet (113), in such a way as to allow for a circulation of fluid in the micro-chambers (111.0) of the first chamber (110).

3. Filtration device (100) according to one of the preceding claims, wherein the micro-chambers (111.0) have a measurement of length and a measurement of width with respect to a direction of circulation of the fluid each between $500\mu$m and $550\mu$m.

4. Filtration device (100) according to one of the preceding claims, wherein the micro-channels (111.2) have a measurement of length with respect to a direction of circulation of the fluid between $700\mu$m and $750\mu$m and a measurement of width with respect to a direction of circulation of the fluid between $200\mu$m and $250\mu$m.

5. Filtration device (100) according to one of the preceding claims, wherein the micro-chambers (111.0) include an inlet zone (111.5) and an outlet zone (111.6), with the microwalls (111.3) comprising angular zones (117) on either side of the inlet zone (115) and of the outlet zone (116) of at least one chamber (110), with the angular zones (117) having a measurement of length with respect to a direction of circulation of the fluid between $100\mu$m and $120\mu$m in such a way as to define, on either side of the inlet zone (111.5) of said chamber (111.0) partially protected zones.

6. Filtration device (100) according to one of the preceding claims, wherein the first chamber (110) has a culture surface in a ratio with a global surface of the bottom wall (111) between 90% and 110%.

7. Filtration device (100) according to one of the preceding claims, wherein the first chamber (110) has a culture surface in a ratio with an available global volume of fluid of the first chamber (110) between 4/mm and 6/mm.

8. Filtration device (100) according to one of the preceding claims, wherein the second chamber (120) comprises a top wall (121) having a set of microstructures identical to that of the bottom wall (111) of the first chamber (110).

9. Filtration device (100) according to one of the preceding claims, wherein the membrane (130) is made of a material suited for allowing a culture of cells on the membrane (130).

10. Filtration device (100) according to one of the preceding claims, further comprising a means for maintaining (160) having a locked configuration wherein are maintained integral on one hand the first block (101) and the membrane (130), on the other hand the membrane (130) and the second block, and an unlocked configuration, wherein the block (101) and the membrane (130) can be separated from one another and/or wherein the membrane (130) and the second block (102) can be separated from one another, with the means for maintaining (160) able to be switched from the locked configuration to the unlocked configuration and inversely.

11. Filtration system, **characterised in that** it comprises

   - a filtration device (100) according to one of claims 1 to 10, and
   - a fluid circuit (300) comprising a circulation pipe (310) provided with a means for circulation (320) and connected to the first and second openings (122, 123) in the second chamber (120).

12. Filtration system as claimed in the preceding claim, wherein the filtration device (100) comprises

   - a fluid inlet (112) in the first chamber (110) connected to at least one portion of the micro-channels (111.2),
   - a fluid outlet (113) in the first chamber (110) connected to at least one portion of the micro-channels (111.2).
   with the micro-channels forming a network connecting the fluid inlet (112) to each micro-chamber (111.0) and each micro-chamber (111.0) to the fluid outlet (113), in such a way as to allow for a circulation of fluid in the micro-chambers (111.0) of the first chamber (110).
   with the device further comprising a fluid circuit (200) comprising a circulation pipe (210) provided with a means for circulation (220) and connected to the fluid inlet (112) and outlet (113) in the first chamber (110).

13. Filtration system according to one of claims 11 to 12, wherein the first circuit (200) further comprises a means for controlling (230) in order to control a fluid pressure in the first chamber (110).

14. Filtration system according to one of claims 11 to 13, wherein the second circuit (300) further comprises a means for controlling (330) in order to control a fluid pressure in the second chamber (120).

15. Filtration system **characterised in that** it comprises several filtration devices (100) according to one of claims 1 to 10 connected in series by circulation circuits.

Fig. 1

Fig. 2

Fig. 3

Fig. 4

Fig. 5

Fig. 6

Fig. 7

Fig. 8a

Fig. 8b

Fig. 9

Fig. 10

EP 2 480 654 B1

Fig. 11

Fig. 12

Fig. 13

Fig. 14

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 6197575 B
- US 7048856 B
- WO 2004020590 A
- FR 0954288